(19) Europäisches Patentamt
European Patent Office
Office européen des brevets

(11) Publication number: 0 194 046 A1

(12) **EUROPEAN PATENT APPLICATION**

(21) Application number: 86300885.0

(22) Date of filing: 10.02.86

(51) Int. Cl.[4]: C 07 D 211/90, C 07 D 401/12, A 61 K 31/44

(30) Priority: 11.02.85 GB 8503426

(43) Date of publication of application: 10.09.86 Bulletin 86/37

(84) Designated Contracting States: AT BE CH DE FR GB IT LI LU NL SE

(71) Applicant: IMPERIAL CHEMICAL INDUSTRIES PLC, Imperial Chemical House Millbank, London SW1P 3JF (GB)

(72) Inventor: Hargreaves, Rodney Brian, 42 Deva Close, Poynton Cheshire (GB)
Inventor: McLoughlin, Bernard Joseph, 7 Pexhill Drive, Macclesfield Cheshire (GB)
Inventor: Mills, Stuart Dennett, 17 Harrington Drive, Gawsworth Macclesfield, Sk11 9RD (GB)
Inventor: Taylor, Melvin Joseph, 7 Kenmore Road, Sale Cheshire (GB)

(74) Representative: Slatcher, Reginald Peter et al, Imperial Chemical Industries PLC Legal Department: Patents P.O. Box 6 Bessemer Road, Welwyn Garden City AL7 1HD (GB)

(54) **Dihydropyridine alkanol amines, process for their preparation and pharmaceutical compositions containing them.**

(57) A dihydropyridine of the formula:

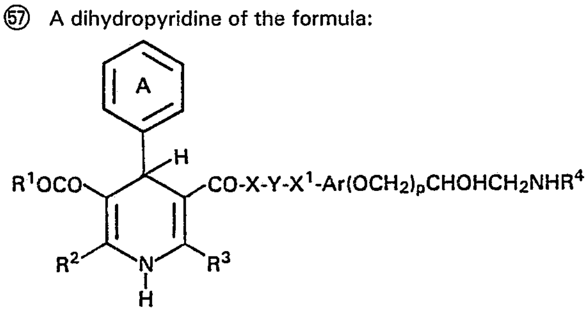

wherein $R^1$ is alkyl or alkoxyalkyl, wherein $R^2$ and $R^3$ each is alkyl, wherein $R^4$ is alpha-branched-chain alkyl, hydroxyalkyl, arylalkyl or aryloxyalkyl, or acylaminoalkyl, wherein benzene ring A bears one or more substituents selected from halogeno, cyano, nitro, trifluoromethyl and alkyl or bears the substituent =N-O-N= attached to the 2- and 3-positions, wherein Ar is phenylene, naphthylene, tetrahydronaphthylene, indanylene or pyridylene which is unsubstituted or which bears one or more substituents; wherein p is 0 or 1;
wherein X is -O- or -NH-;
wherein $X^1$ is a direct link or is -O-, -S-, -NH- or $-NHSO_2-$;
and wherein Y is straight- or branched-chain alkylene which may optionally be interrupted by one or two groups selected from oxygen, sulphur, imino, substituted imino, phenylene, substituted phenylene, pyridylene, cycloalkylene, 1,4-piperazinediyl, 1,3- or 1,4-piperidinediyl and amido groups;
or an acid-addition salt thereof, processes for their manufacture and pharmaceutical compositions containing them. The compound possess either beta-adrenergic blocking or calcium ion slow channel blocking properties, or both such properties, and may be used in the treatment of hypertension.

EP 0 194 046 A1

TITLE MODIFIED
see front page



TITLE MODIFIED
see front page

PYRIDINE DERIVATIVES

This invention relates to new pyridine derivatives and more particularly it relates to new dihydropyridine derivatives which possess antihypertensive properties.

Many 2,6-dialkyl-4-aryl-1,4-dihydropyridine-3,5-dicarboxylate derivatives are known which inhibit the movement of calcium ions in the cardiovascular system of warm-blooded animals, and which thereby produce an antihypertensive effect. The most-used of these is nifedipine, which is dimethyl 1,4-dihydro-2,6-dimethyl-4-o-nitrophenylpyridine-3,5-dicarboxylate.

Also known are many 1-aryloxy-3-amino-propan-2-ol derivatives which possess beta-adrenergic receptor blocking properties and which also produce an antihypertensive effect. Two of the most-used of these are propranolol and atenolol, which are respectively 1-(naphth-1-yloxy)- and 1-p-carbamoylmethylphenoxy-3-isopropylaminopropan-2-ol.

The only described attempt to combine these two types of chemical structure into one molecule is reported by Merck workers in the Journal of Medicinal Chemistry, 1981, Vol. 24, pages 628 to 631, in which a 3-amino-2-hydroxypropoxy substituent was introduced into the 4-aryl substituent of a 4-aryl-1,4-dihydropyridine derivative, without much success in producing a compound with antihypertensive activity of the type sought by the authors.

We have now found that compounds which do possess useful antihypertensive activity may be obtained by suitably combining a 3-aryloxy-2-hydroxypropylamino moiety with a 1,4-dihydropyridine moiety.

According to the present invention there is provided a dihydropyridine of the formula:

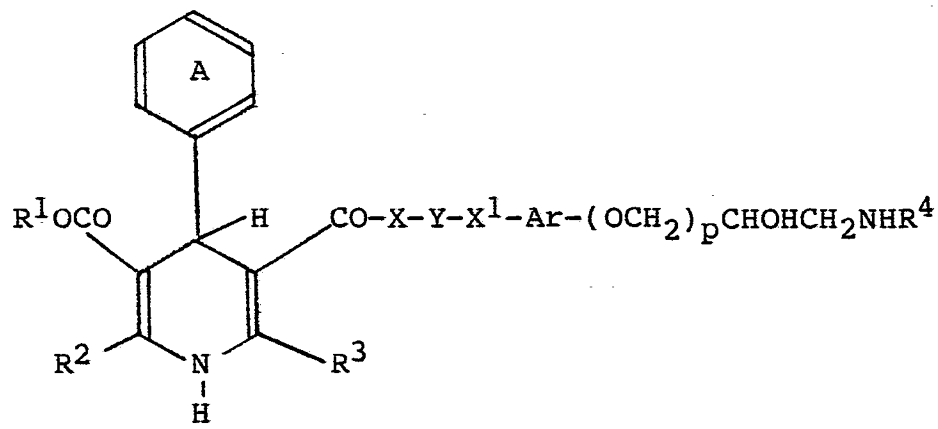

wherein $R^1$ is alkyl or alkoxyalkyl each of up to 6 carbon atoms: wherein $R^2$ and $R^3$, which may be the same or different, each is alkyl of up to 6 carbon atoms; wherein $R^4$ is alpha-branched-chain alkyl or hydroxyalkyl each of up to 6 carbon toms, or alpha-branched-chain arylalkyl or aryloxyalkyl each of up to 12 carbon atoms, or acylaminoalkyl wherein the acyl is of up to 10 carbon atoms and the alkyl is of up to 6 carbon atoms; wherein benzene ring A bears one or more substituents selected from halogeno, cyano, nitro, trifluoromethyl and alkyl of up to 6 carbon atoms or bears the substituent

=N-O-N=

attached to the 2- and 3-positions (that is, to form a benzo-2,1,3-oxadiazole nucleus); wherein Ar is phenylene, naphthylene, tetrahydronaphthylene, indanylene or pyridylene which is unsubstituted or which bears one or more substituents selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;

wherein p is 0 or 1;
wherein X is -O- or -NH-;
wherein $X^1$ is a direct link or is -O-, -S-, -NH or $-NHSO_2-$;
and wherein Y is straight-or branched-chain alkylene of 1 to 12 carbon atoms which may optionally be interrupted by one or two groups selected from oxygen (-O-), sulphur (-S-), imino and substituted imino ($-NR^5$ wherein $R^5$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,3- or 1,4-piperidinediyl and amido (-CONH-or -NHCO-) groups;

or an acid-addition salt thereof.

It will be observed that the dihydropyridine derivative of the invention possesses at least two asymmetric carbon atoms, namely the carbon atom of the -CHOH- group in the alkanolamine chain, and the carbon atom at the 4-position of the dihydropyridine nucleus, and it can therefore exist in racemic and optically-active forms. It is to be understood that this invention encompasses the racemic form of the dihydropyridine derivative and any optically-active form which possesses antihypertensive activity, it being a matter of common general knowledge how a racemic compound may be resolved into optically-active forms, and how the antihypertensive activity of these forms may be determined. It is further to be understood that beta-adrenergic blocking activity usually predominates in that optically-active form which has the "S" absolute configuration of the said -CHOH- group in the alkanolamine chain when p is 1 and the "R" absolute configuration when p is 0.

A suitable value for $R^1$, $R^2$, $R^3$, $R^5$ or a substituent in benzene ring A or Ar when it is alkyl is, for example, methyl, ethyl or isopropyl.

A suitable value for $R^1$ when it is alkoxyalkyl is, for example, methoxyethyl, ethoxyethyl or propoxyethyl.

A suitable value for $R^4$ is, for example, isopropyl, s-butyl, t-butyl, 2-hydroxy-1-methylethyl, 2-hydroxy-1,1-dimethylethyl, 1-methyl-3-phenylpropyl, 1-methyl-2-phenoxyethyl or 2-isobutyramidoethyl.

A suitable halogeno substituent in the benzene ring A or in Ar is, for example fluoro, chloro or bromo.

A suitable value for the alkenyl, alkoxy, alkenyloxy, alkoxyalkoxy, alkylthio, carbamoylalkyl or alkanoylamino substituent in Ar is, for example, allyl, methoxy, ethoxy, isopropoxy, allyloxy, methoxyethoxy, methylthio, carbamoylmethyl or acetamido.

A suitable value for $R^5$ when it is alkanoyl, or for an alkanoyl substituent in Ar is, for example,formyl, acetyl or benzoyl.

A suitable value for $R^5$ when it is aralkyl, or for an aralkyl substituent in Ar is, for example, benzyl.

A suitable value for Y is, for example, straight-chain alkylene of the formula $(-CH_2)_n-$, wherein n is an integer from 1 to 12;

or $-(CH_2)_mC(CH_3)_2-$;

or $-(CH_2)_m-NH-(CH_2)_n-$

$-(CH_2)_m-N(CH_3)-(CH_2)_n-$

$-(CH_2)_m-O-(CH_2)_n-$

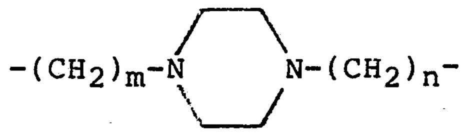

wherein m and n, which may be the same or different, each is 2,3,4 or 5;

or 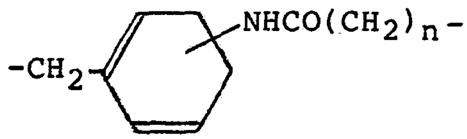

$-(CH_2)_mNHCO(CH_2)_n-$

wherein m is 2, 3, 4 or 5 and n is 1, 2 or 3;

or 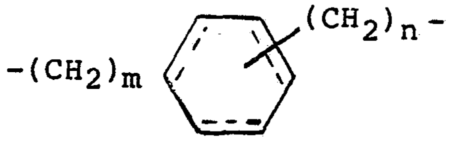 or 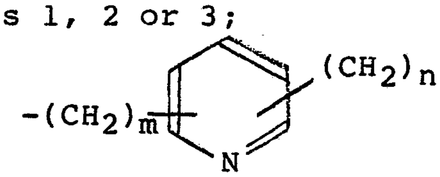

and wherein m and n, which may be the same or different, each is 1, 2, 3 or 4 and wherein the double bonds in the carbocyclic ring are optional (that is, cyclohexylene- or phenylene- bis-alkylene).

A preferred dihydropyridine derivative of the invention has the formula stated above wherein $R^1$ is methyl or ethyl, $R^2$ and $R^3$ are both methyl, $R^4$ is isopropyl, t-butyl, 2-hydroxy-1-methylethyl, 2-hydroxy-1,1-dimethylethyl or 2-isobutyramidoethyl, benzene ring A is 3-nitrophenyl, 2-chlorophenyl or 2,3-dichlorophenyl, Ar is 1,4-phenylene or 1,2-phenylene which may contain a chloro or methoxy substituent, or is 1,5-naphthalene, p is 0 or 1, X is -O-, Y is $-(CH_2)_n-$ which may optionally be interrupted by -O- and/or -NHCO-, and $X^1$ is a direct link or -O-.

A particularly preferred dihydropyridine has the last-mentioned definition wherein $-YX^1-$ is $-(CH_2)_n-O-$ or $-(CH_2)_mNHCOCH_2-$ wherein n is 2, 3, 4, 5, 6 or 7 and m is 2, 3, 4, 5 or 6, or is $-(CH_2)_2-O-(CH_2)_2NHCOCH_2-$.

A suitable acid-addition salt of a dihydropyridine derivative of the invention is, for example, a salt derived from an inorganic acid, for example a hydrochloride, hydrobromide, phosphate or sulphate, or a salt derived from an organic acid, for

example an oxalate, lactate, succinate, tartrate, acetate, salicylate, citrate, benzoate, beta-naphthoate or adipate.

Specific dihydropyridine derivatives of the invention are hereinafter described in the Examples. Of these, preferred compounds are:-

3-[*p*-(2-hydroxy-3-isopropylaminopropoxy)-phenylacetamido]propyl 5-ethoxycarbonyl-1,4-dihydro-2,6-dimethyl-4-*m*-nitrophenylpyridine-3-carboxylate;

3-[*p*-(2-hydroxy-3-isopropylaminopropoxy)-phenoxy]propyl 5-ethoxycarbonyl-1,4-dihydro-2,6-dimethyl-4-*m*-nitrophenylpyridine-3-carboxylate and the corresponding 4-[*p*-(2-hydroxy-3-isopropylaminopropoxy)-phenoxy]butyl, 5-[*p*-(2-hydroxy-3-isopropylamino-propoxy)phenoxy]pentyl, 6-[*p*-(2-hydroxy-3-isopropyl-aminopropoxy)phenoxy]hexyl and 5-[4-(2-hydroxy-3-isopropylaminopropoxy)-3-methoxyphenoxy]pentyl esters;

3-[5-(2-hydroxy-3-isopropylaminopropoxy)-naphth-1-yloxy]propyl 1,4-dihydro-5-methoxycarbonyl-2,6-dimethyl-4-*m*-nitrophenylpyridine-3-carboxylate and the corresponding 5-[5-(2-hydroxy-3-isopropylaminopropoxy)-naphth-1-yloxy]pentyl ester;

4-[5-(2-hydroxy-3-isopropylaminopropoxy)-naphth-1-yloxy]butyl 5-ethoxycarbonyl-1,4-dihydro-2,6-dimethyl-4-*m*-nitrophenylpyridine-3-carboxylate;

2-[*p*-(1-hydroxy-2-isopropylaminoethyl)-phenoxy]ethyl 5-ethoxycarbonyl-1,4-dihydro-2,6-dimethyl-4-*m*-nitrophenylpyridine-3-carboxylate and the corresponding 3-[*p*-(1-hydroxy-2-isopropylaminoethyl)-phenoxy]propyl ester;

4-[*p*-(1-hydroxy-2-isopropylaminoethyl)-phenoxy]butyl 1,4-dihydro-5-methoxycarbonyl-2,6-dimethyl-4-*m*-nitrophenylpyridine-3-carboxylate; and

1-[*p*-(2-hydroxy-3-isopropylaminopropoxy)-benzyl]piperidin-3-yl 1,4-dihydro-5-methoxycarbonyl-2,6-dimethyl-4-*m*-nitrophenylpyridine-3-carboxylate.

The dihydropyridine derivatives of the invention may be manufactured by any chemical process known to be useful for the manufacture of chemically-analogous compounds.

One preferred process for the manufacture of a dihydropyridine derivative of the invention comprises the reaction of an epoxide of the formula:-

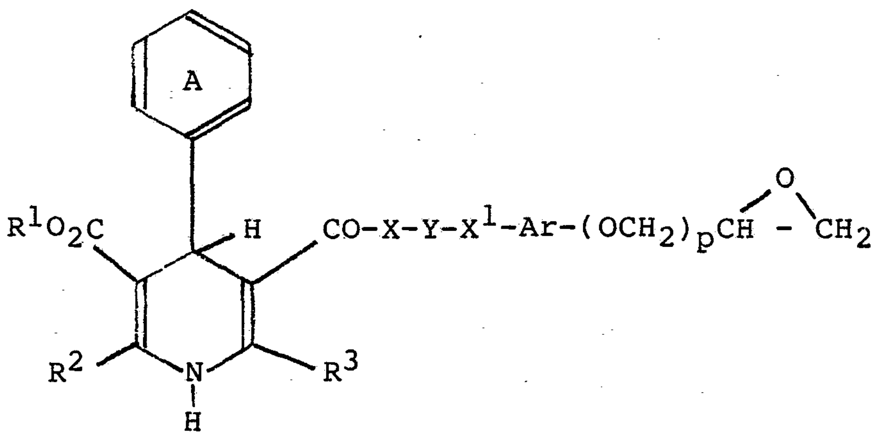

wherein A, $R^1$, $R^2$, $R^3$, X, $X^1$, Y, Ar and p have the meanings stated above, with an amine of the formula $R^4NH_2$ wherein $R^4$ has the meaning stated above, or when p is 0, the reaction of said amine with a haloketone of the formula

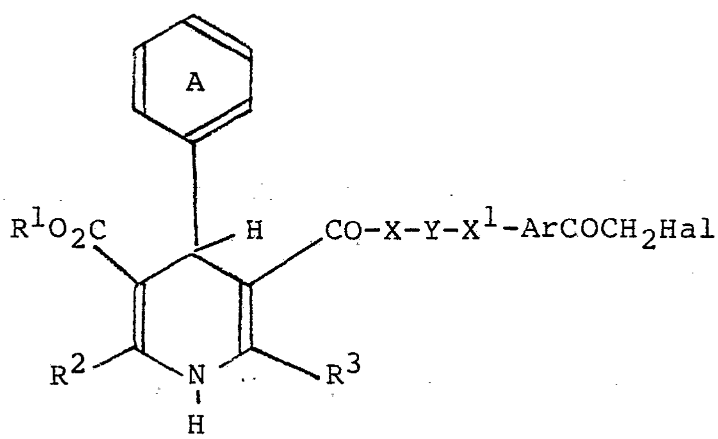

wherein A, $R^1$, $R^2$, $R^3$, X, $X^1$, Y and Ar have the meanings stated above and wherein Hal stands for a halogeno

group, for example bromo, followed by reduction, for example with sodium borohydride, of the aminoketone thus obtained.

The reaction may be carried out in an alcoholic diluent or solvent, for example in isopropanol, at a temperature of up to the boiling point of said diluent or solvent.

A second preferred process for the manufacture of a dihydropyridine derivative of the invention wherein the group -Y- is alkylene interrupted as stated above comprises joining the two parts of the molecule at the point of interruption of Y. Thus, for example, when Y is alkylene interrupted by an amido group -NHCO-, the process comprises the reaction of an amine of the formula:-

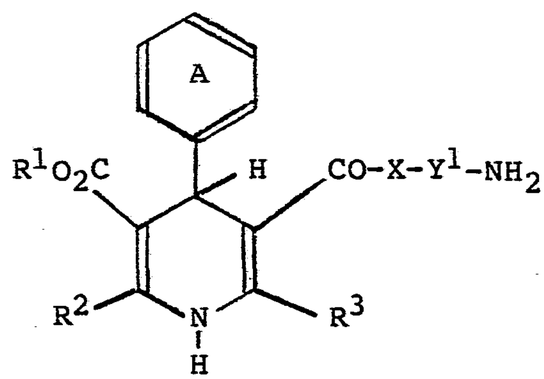

wherein A, $R^1$, $R^2$, $R^3$ and X have the meanings stated above, with an acid of the formula:-

$$HO_2C-Y^2-X^1-Ar-(OCH_2)_pCHOHCH_2NHR^4$$

wherein $X^1$, Ar, p and $R^4$ have the meanings stated above, or with an activated derivative thereof, and wherein $Y^1$ and $Y^2$ are such that $-Y^1-NHCO-Y^2-$ has the same meaning as stated above for Y.

A third process for the manufacture of a dihydropyridine derivative of the invention comprises the reaction of an acid of the formula:-

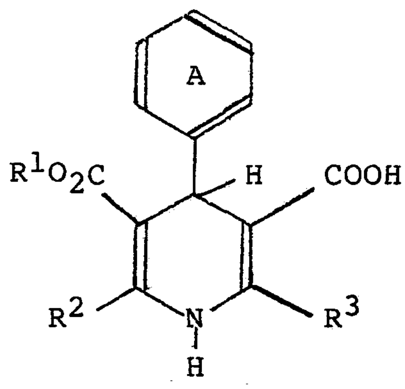

wherein A, $R^1$, $R^2$ and $R^3$ have the meanings stated above, or an activated derivative thereof, with an alcohol or amine of the formula:-

$$H\text{-}X\text{-}Y\text{-}X^1\text{-}Ar(OCH_2)_pCHOHCH_2NHR^4$$

wherein $R^4$, Ar, p, X, $X^1$ and Y have the meanings stated above.

A fourth process for the manufacture of a dihydropyridine derivative of the invention wherein $X^1$ is other than a direct link comprises the reaction of a compound of the formula:-

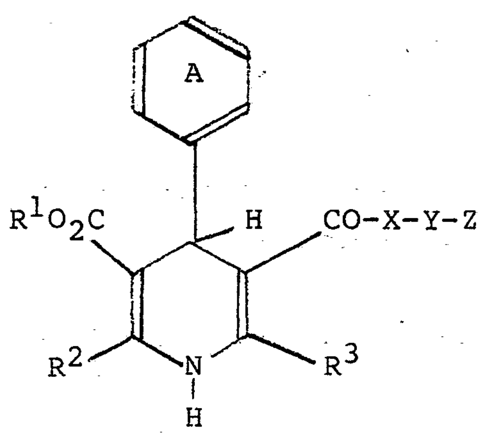

wherein A, $R^1$, $R^2$, $R^3$, X and Y have the meanings stated above and wherein Z stands for a displaceable group, with a compound of the formula:-

$$H-X^1-Ar(OCH_2)_pCHOHCH_2NHR^4$$

wherein $X^1$,Ar,p and $R^4$ have the meanings stated above.

A suitable value for Z is, for example, a halogeno group, for example a bromo or chloro group, or a sulphonyloxy group, for example a methanesulphonyloxy or p-toluenesulphonyloxy group.

A fifth process for the manufacture of a dihydropyridine of the invention comprises the reaction of an aldehyde of the formula

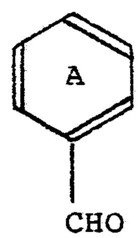

wherein A has the meaning stated above, an aminocrotonate of the formula

$$R^2-C(NH_2)=CH-CO_2R^1$$

wherein $R^1$ and $R^2$ have the meanings stated above and a ketoacid derivative of the formula

$$R^3COCH_2CO-X-Y-X^1-Ar(OCH_2)_pCHOHCH_2NHR^4$$

wherein Ar, p, $R^3$, $R^4$, X and Y have the meanings stated above.

This process may be carried out in a diluent or solvent at an elevated temperature, conditions conventionally used for the Hantszch synthesis of dihydropyridines.

As stated above, the dihydropyridine derivatives of the invention possess antihypertensive activity. This may be demonstrated by the ability of the compound to reduce the blood pressure of a spontaneously hypertensive rat, or of a rat made hypertensive by treatment with deoxycorticosterone acetate, or of a dog made hypertensive by the Goldblatt technique of unilateral nephrectomy and clipping of the contralateral kidney. These are all standard tests used to demonstrate antihypertensive effects of medicaments.

Some of the dihydropyridine derivatives of the invention possess beta-adrenergic blocking properties, some of them possess calcium ion slow-channel blocking properties and some of them possess both such activities. A preferred dihydropyridine derivative of the invention possesses both such activities. Beta-adrenergic blocking activity may be demonstrated *in vivo* by the ability of the compound to inhibit isoprenaline-induced tachycardia in a rat or cat, or *in vitro* by shifting to the right the dose- response curve of a guinea pig atrium to isoprenaline. Calcium ion slow channel blocking activity may be demonstrated *in vitro* by the ability of the compound to reduce spontaneous contraction in a rat portal vein preparation. These also are all standard tests used to demonstrate the stated activities.

Because of the beta-adrenergic blocking and/or calcium slow channel blocking properties a dihydropyridine of the invention may also be useful in the treatment of heart diseases such as angina pectoris and cardiac arrhythmias.

At doses of a dihydropyridine derivative which produce effective antihypertensive activity in a rat or dog no symptom of toxicity is apparent.

The dihydropyridine derivative of the invention may be administered to warm-blooded animals, including man, in the form of a pharmaceutical composition comprising as active ingredient at least one dihydropyridine derivative of the invention, or an acid-addition salt thereof, in association with a pharmaceutically-acceptable diluent or carrier therefor.

A suitable composition is, for example, a tablet, capsule, aqueous or oily solution or suspension, dispersible powder, spray or aerosol formulation.

The pharmaceutical composition may contain, in addition to the dihydropyridine derivative of the invention, one or more drugs selected from sedatives, for example phenobarbitone, meprobamate, chloropromazine and the benzodiazepine sedative drugs, for example chlordiazepoxide and diazepam; vasodilators, for example glyceryl trinitrate, pentaerythritol tetranitrate, isosorbide dinitrate and hydralazine; diuretics, for example chlorthalidone, bendrofluazide, hydrochlorothiazide and chlorothiazide; other antihypertensive agents, for example reserpine, bethanidine and guanethidine; cardiac membrane stabilising agents, for example quinidine; agents used in the treatment of Parkinson's disease and other tremors, for example benzhexol; cardiotonic agents, for example digitalis preparations; and alpha-adrenergic blocking agents, for example phentolamine.

When used for the treatment of heart diseases, for example angina pectoris and cardiac arrhythmias, or for the treatment of hypertension in man, it is expected that the dihydropyridine derivative would be given to

man at a total oral dose of between 20 mg. and 600 mg. daily, or at an intravenous dose of between 1 mg. and 20 mg.

Preferred oral dosage forms are tablets or capsules containing between 10 and 100 mg., and preferably 10 mg. or 50 mg., of active ingredient. Preferred intravenous dosage forms are sterile solutions of the dihydropyridine derivative or of a non- toxic acid-addition salt thereof, containing between 0.05% and 1% w/v of active ingredient, and more particularly containing 0.1% w/v of active ingredient.

The invention is illustrated but not limited by the following Examples:-

Example 1

A mixture of 3-(*p*-hydroxyphenylacetamido)-propyl 5-ethoxycarbonyl-1,4-dihydro-2,6-dimethyl-4-*m*-nitrophenylpyridine-3-carboxylate (2.7 g.), epichlorohydrin (3.0 ml.), ethanol (50 ml.) and aqueous N-sodium hydroxide solution (5 ml.) was kept at laboratory temperature for 18 hours and then evaporated to dryness, and the residue was partitioned between ethyl acetate and water. The organic layer was separated, washed with water, dried over magnesium sulphate and evaporated to dryness under reduced pressure, and the residue was dissolved in a mixture of ethanol (5 ml.) and isopropylamine (3 ml.). The mixture was heated under reflux for 4 hours, kept at laboratory temperature for 20 hours and then evaporated to dryness under reduced pressure. The residue was shaken with water (15 ml.) and ethyl acetate (15 ml.) and the organic layer was separated, washed with water, dried over magnesium sulphate and evaporated to dryness under reduced pressure. The residue was purified by flash chromatography on a silica gel column (Merck 9385) using a 4:1 v/v mixture of methylene chloride and methanol as

eluant. There was thus obtained as an oil 3-[p-(2-hydroxy-3-isopropylaminopropoxy)phenylacetamido]propyl 5-ethoxycarbonyl-1,4-dihydro-2,6-dimethyl-4-m-nitrophenylpyridine-3-carboxylate hemihydrate, the structure of which was confirmed by elemental analysis and proton magnetic resonance spectroscopy.

Elemental Analysis

Found: C,60.8%; H,6.7%; N,8.5%. $C_{34}H_{44}N_4O_9 . \frac{1}{2}H_2O$ requires: C,60.9%; H,6.9%; N,8.4%.

Proton magnetic resonance spectrum (in $CD_3SOCD_3$)

| Shift(δ) | Type | No of Protons | Assignment |
|---|---|---|---|
| 0.97 | doublet | 6 | $(CH_3)_2CH-$ |
| 1.1 | triplet | 3 | $CH_3(CH_2OCO)$ |
| 1.65 | multiplet | 2 | $C-(CH_2)-C$ |
| 2.3 | singlet | 6 | pyridine 2,6-methyl |
| 2.5-3.1 | complex | 7 | $CH_2$ and CH adjacent NH, NH and OH |
| 3.3 | singlet | 2 | $COCH_2$-phenyl |
| 3.9-4.1 | complex | 7 | $CH_2$ and CH adjacent O |
| 5.0 | singlet | 1 | pyridine 4H |
| 6.7-8.0 | complex | 9 | 8 aromatic, pyridine NH |
| 8.8 | singlet | 1 | amide NH |

The carboxylate used as starting material was obtained as follows:-

A stirred mixture of 5-ethoxycarbonyl-1,4-dihydro-2,6-dimethyl-4-m-nitrophenylpyridine-3-carboxylic acid (34.6 g.), N-(3-bromopropyl)phthalimide

(26.8 g.), potassium carbonate (50 g.) and acetone (500 ml.) was heated under reflux for 18 hours, the solvent was removed by evaporation and the residue was partitioned between ethyl acetate and water. The organic layer was separated, washed with water, dried over magnesium sulphate and evaporated to dryness, and the residue was crystallised from toluene. There was thus obtained 3-phthalimidopropyl 5-ethoxycarbonyl-1,4-dihydro-2,6-dimethyl-4-m-nitrophenylpyridine-3-carboxylate, m.p.78-80°C.

A mixture of the above compound (35.7 g.), hydrazine hydrate (22.5 ml.) and ethanol (500 ml.) was heated under reflux for 1 hour, cooled and filtered and the filtrate was evaporated to dryness. The residue was stirred with ethyl acetate (300 ml) for 20 hours, the mixture was filtered and the filtrate was evaporated to dryness.

A mixture of the solid 3-aminopropyl ester thus obtained (4.0 g.), p-hydroxyphenylacetic acid (1.5 g.),1-hydroxybenzotriazole (1.5 g.), dicyclohexylcarbodiimide (2.5 g.) and methylene chloride (150 ml.) was stirred at laboratory temperature for 18 hours and then filtered, and the filtrate was washed with water and then with saturated aqueous sodium bicarbonate solution, dried over magnesium sulphate and evaporated to dryness. The residue was stirred with ethyl acetate for 2 hours, the mixture was filtered and the filtrate was evaporated to dryness. There was thus obtained as residue 3-(p-hydroxyphenylacetamido)propyl 5-ethoxycarbonyl-1,4-dihydro-2,6-dimethyl-4-m-nitrophenylpyridine-3-carboxylate which was used without further purification.

Example 2

The process described in Example 1 was repeated using the appropriate pyridine-3-carboxylate,

epichlorohydrin and the appropriate amine as starting materials, and there were thus obtained as oils the compounds described in the following tables, the structures of all of which were confirmed by elemental analysis, mass spectroscopy and proton magnetic resonance spectroscopy.

TABLE I

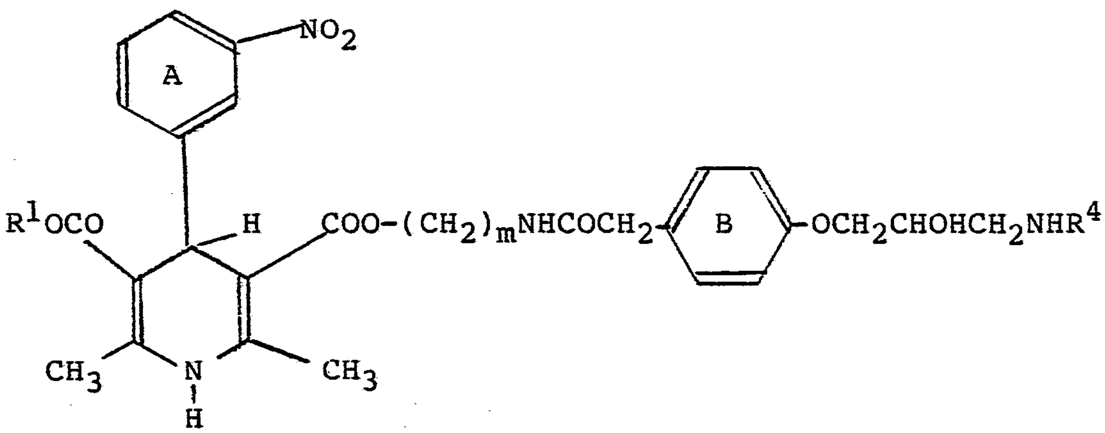

| $R^1$ | m | $R^4$ |
|---|---|---|
| ethyl | 2 | isopropyl |
| ethyl | 2 | t-butyl |
| methyl | 3 | isopropyl |
| ethyl | 3 | isopropyl (Note 1) |
| methyl | 3 | t-butyl |
| ethyl | 3 | 2-hydroxy-1,1-dimethylethyl |
| methyl | 4 | isopropyl |
| ethyl | 4 | isopropyl |
| methyl | 4 | t-butyl |
| ethyl | 4 | t-butyl |
| ethyl | 4 | 2-hydroxy-1,1-dimethylethyl |
| ethyl | 4 | 2-hydroxy-1-methylethyl |
| ethyl | (Note 2) | isopropyl |
| ethyl | 6 | isopropyl |
| methyl | 3 | isopropyl (Note 3) |
| ethyl | 3 | isopropyl (Note 4) |

Note 1 The 2-hydroxy-3-isopropylaminopropoxy group is in the ortho-, not para-, position of the benzene ring B.

Note 2 The linking group is $-(CH_2)_2-O-(CH_2)_2$ in place of $-(CH_2)_m$.

Note 3 Ring A is 2,3-dichlorophenyl in place of 3-nitrophenyl.

Note 4 Ring B has an additional chloro substituent ortho- to the 2-hydroxy-3-isopropylaminopropoxy group.

TABLE 2

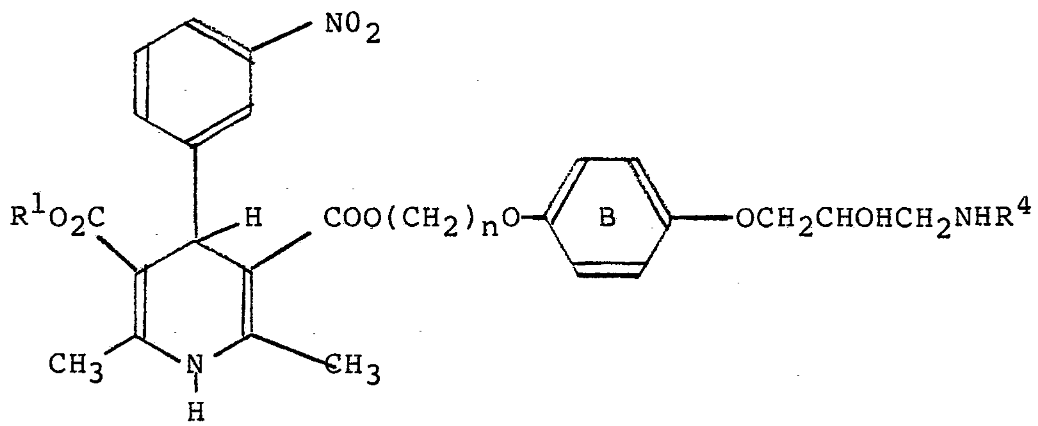

| $R^1$ | n | $R^4$ |
|---|---|---|
| ethyl | 3 | isopropyl |
| methyl | 5 | isopropyl |
| ethyl | 5 | isopropyl |
| ethyl | 5 | isopropyl (Note 5) |
| ethyl | 5 | t-butyl (Note 5) |
| ethyl | 6 | isopropyl |
| ethyl | 7 | isopropyl |

Note 5 Ring B has an additional methoxy substituent ortho- to the 2-hydroxy-3-alkylaminopropoxy group.

The starting materials for the compounds in Table I were obtained by a similar process to that described in the second part of Example 1 using the appropriate N-(omega-bromoalkyl)phthalimide in place of N-(3-bromopropyl)phthalimide and the appropriate 5-alkoxycarbonyldihydropyridine. 3-Phthalimidopropyl 1,4-dihydro-5-methoxycarbonyl-2,6-dimethyl-4-m-nitrophenylpyridine-3-carboxylate has m.p. 158-164°C. after crystallisation from ethyl acetate, and the corresponding 4-phthalimidobutyl ester has m.p. 154-156°C. after crystallisation from toluene. In the case of the compound described under Note 2 the intermediate used was N-[2-(2-methanesulphonyloxyethoxy)-ethyl]phthalimide.

The starting materials for the compounds in Table 2 were obtained by the reaction of 5-ethoxycarbonyl-1,4-dihydro-2,6-dimethyl-4-m-nitrophenylpyridine-3-carboxylic acid with the appropriate p-(omega-bromoalkoxy)phenol under similar conditions to the reaction of said carboxylic acid with the bromoalkylphthalimide.

In the case of the compound described under Note 5 the intermediate was obtained as follows:-

p-5-Bromopentyloxyphenol (18.2 g.) was added to a stirred solution of benzoyl peroxide (22.7 g.) in toluene (450 ml.) at laboratory temperature and the mixture was stirred and heated at 90°C. for 6 hours and then at laboratory temperature for 15 hours, and evaporated to dryness under reduced pressure. The residue was purified by flash chromatography on a silica gel (Merck 9385) column using as eluent methylene chloride containing increasing amounts of methanol, up to 1.5% by volume.

Methyl iodide (0.94 g.) was added to a stirred mixture of the 4-(5-bromopentyloxy)-2-hydroxyphenyl benzoate thus obtained (0.5 g.), potassium carbonate (0.18 g.) and acetone (50 ml.) and the mixture was stirred at laboratory temperature for 48 hours and filtered. The filtrate was evaporated to dryness and the residue was purified by flash chromatography on a silica gel (Merck 9385) column using methylene chloride as eluent.

A mixture of the 4-(5-bromopentyloxy)-2-methoxyphenyl benzoate thus obtained (0.5 g.), 5-ethoxycarbonyl-1,4-dihydro-2,6-dimethyl-4-m-nitrophenyl-pyridine-3-carboxylic acid (0.44 g.), potassium carbonate (0.18 g.) and acetone (50 ml.) was stirred at laboratory temperature for 90 minutes, heated at 90°C. for 12 hours, cooled and evaporated to dryness. The residue was purifed by flash chromatography on a silica gel (Merck 9385) column using methylene chloride as eluent. Aqueous 2N-sodium hydroxide solution (1.9 ml.) was added dropwise to a stirred solution of the benzoate ester thus obtained (0.5 g.) in methanol (30 ml.) and the mixture was stirred for 45 minutes, acidified to pH 4 with concentrated aqueous hydrochloric acid and evaporated to dryness. The residue was partitioned between methylene chloride and aqueous 10% sodium bicarbonate solution and the organic layer was separated, washed with water, dried and evaporated to dryness. The residue was purified by flash chromatography on a silica gel column using as eluent methylene chloride containing increasing amounts of methanol up to 10% by volume. There was thus obtained as an oil 5-(4-hydroxy-3-methoxyphenoxy)pentyl 5-ethoxycarbonyl-1,4-dihydro-2,6-dimethyl-4-m-nitrophenylpyridine-3-carboxylate.

Example 3

The process described in Example 1 was repeated using N-p-hydroxyphenylpiperidin-3-yl 1,4-dihydro-5-methoxycarbonyl-2,6-dimethyl-4-m-nitrophenylpyridine-3-carboxylate as starting material. There was thus obtained as an oil N-p-(2-hydroxy-3-isopropylaminopropoxy)benzylpiperidin-3-yl 1,4-dihydro-5-methoxycarbonyl-6-methyl-4-m-nitrophenylpyridine-3-carboxylate hydrate, the structure of which was confirmed by elemental analysis, mass spectroscopy and proton magnetic resonance spectroscopy:

Elemental Analysis

Found: C, 62.5%; H, 6.9%; N, 8.6%.

$C_{34}H_{44}N_4O_8.H_2O$ requires C, 62.5%; H, 7.05%; N, 8.6%.

Mass Spectroscopy Main ion $M^+H$ = 637

Proton magnetic resonance spectroscopy (in CDCl3)

| Shift (δ) | Type | No. of Protons | Assignment |
|---|---|---|---|
| 1.0-1.2 | 2 singlets | 6 | $(\underline{CH_3})_2CH$ |
| 1.3-1.8 | broad | 4 | $CH_2CH_2$ in piperidine |
| 2.3-2.6 | 2 singlets | 6 | pyridine-2,6-methyl |
| 2.1-3.0 | complex | 9 | $\underline{CH_2}N$, $\underline{CH}(CH_3)_2$, OH, NH |
| 3.4-3.5 | singlet | 2 | benzyl $CH_2$ |
| 3.6 | singlet | 3 | $CH_3O$ |
| 4.0 | complex | 3 | $-O\underline{CH_2}-\underline{CH}OH-$ |
| 4.7-5.0 | broad | 1 | CH-O- in piperidine |

| Shift (δ) | Type | No. of protons | Assignment |
|---|---|---|---|
| 5.1 | singlet | 1 | pyridine 4H |
| 5.9 | singlet | 1 | pyridine NH |
| 6.8-7.3 | quartet | 4 | benzyl aromatic |
| 7.3-8.2 | complex | 4 | nitrophenyl aromatic |

The starting material was obtained as follows:-

A solution of p-hydroxybenzyl alcohol (0.62 g.) in dimethylformamide (1 ml.) was added to a stirred solution of piperidin-3-yl 1,4-dihydro-5-methoxycarbonyl-2,6-dimethyl-4-m-nitrophenylpyridine-3-carboxylate (2.25 g.) in dimethylformamide (5 ml.) which was kept under an atmosphere of argon, and the mixture was stirred at laboratory temperature for 1 hour. Further p-hydroxybenzyl alcohol (0.62 g.) in dimethylformamide (1 ml.) was added and the mixture was heated under reflux for 2 hours, cooled and partitioned between diethyl ether and water. The organic layer was washed with water, dried and evaporated to dryness, and the residue was purified by flash chromatography on a silica gel (Merck 9385) column using as eluent methylene chloride containing increasing amounts of methanol, up to 10% by volume. There was thus obtained the desired pyridine 3-carboxylate which was used without further purification.

Example 4

A mixture of 4-[5-(2,3-epoxypropoxy)naphth-1-yloxy]butyl 1,4-dihydro-5-methoxycarbonyl-2,6-dimethyl-4-m-nitrophenylpyridine-3-carboxylate (0.5 g.), isopropylamine (10 ml.) and isopropanol (10 ml.) was heated under reflux for 15 hours and then evaporated to dryness under reduced pressure. The residue was purified by flash chromatography on a silica gel (Merck 9385) column using 90:10:3 v/v mixture of ethyl acetate, methanol and aqueous ammonia solution (specific gravity 0.88) as eluent. There was thus obtained as an oil 4-[5-(2-hydroxy-3-isopropylamino)naphth-1-yloxy]butyl 1,4-dihydro-5-methoxycarbonyl-2,6-dimethyl-4-m-nitrophenyl-pyridine-3-carboxylate, the structure of which was confirmed by elemental analysis and proton magnetic resonance spectroscopy.

Elemental Analysis

Found: C, 63.6%; H, 6.5%; N 6.4%.

$C_{36}H_{43}N_3O_4.H_2O$ requires C, 63.6%; H, 6.67%; N, 6.2%.

Proton magnetic resonance spectrum (in CDCl3)

| Shift (δ) | Type | No. of Protons | Assignment |
|---|---|---|---|
| 1.1-1.2 | 2 singlets | 6 | $(\underline{CH_3})_2CH-$ |
| 1.8-2.0 | complex | 4 | $-OCH_2-(\underline{CH_2})_2-CH_2O-$ |
| 2.3-2.4 | 2 singlets | 6 | pyridine 2,6-methyl |
| 2.4-2.6 | broad | 2 | OH, NH |
| 2.85-3.15 | complex | 3 | $(CH_3)_2\underline{CH}-$, $-\underline{CH_2}NH-$ |

| Shift (δ) | Type | No. of Protons | Assigment |
|---|---|---|---|
| 3.65 | singlet | 3 | $CH_3O$ |
| 4.0-4.3 | complex | 7 | $OCH_2$, CHOH |
| 5.1 | singlet | 1 | pyridine 4H |
| 5.8 | singlet | 1 | pyridine NH |
| 6.75-8.1 | complex | 10 | aromatic |

The epoxide used as starting material was obtained as follows:-

The mixture of naphthalene-1,5-diol (50 g.), 1,4-dibromobutane (6.75 g.), potassium hyroxide (7.0 g.) and ethanol (100 ml.) was heated under reflux for 2.5 hours, cooled and filtered through a filter-aid. The filtrate was evaporated to dryness under reduced pressure and the residue was stirred with a 4:1 v/v mixture of petroleum ether (b.p. 60-80°C.) and ethyl acetate and the mixture was filtered. The filtrate was evaporated to dryness under reduced pressure and the residue was purified by flash chromatography on a silica gel (Merck 9385) column using a 4:1 v/v mixture of petroleum ether and ethyl acetate as eluent.

A mixture of the 5-(4-bromobutoxy)naphth-1-ol thus obtained (5.0 g.), ethanol (40 ml.), sodium hydroxide (0.68 g.) and epichlorohydrin (12.54 g.) was stirred at laboratory temperature for 15 hours and then evaporated to dryness under reduced pressure. The residue was partitioned between water and ethyl acetate and the organic layer was separated, washed with water,

dried and evaporated to dryness. The residue was stirred with petroleum ether (b.p. 60-80°C.) and the mixture was filtered.

A mixture of the 1-(2,3-epoxypropoxy)-5-(4-bromobutoxy)naphthalene thus obtained (1.5 g.), 1,4-dihydro-5-methoxycarbonyl-2,6-dimethyl-4-m-nitrophenyl-pyridine-3-carboxylic acid (1.42 g), potassium carbonate (2.05) and acetone (40 ml.) was heated under reflux for 6 hours and then evaporated to dryness under reduced pressure. The residue was purified by flash chromatography on a silica gel (Merck 9385) column using a 100:3 v/v mixture of ethyl acetate and triethylamine as eluent. There was thus obtained the desired epoxide which was used without further purification.

The process described in the first paragraph above was repeated using the appropriate epoxide and the appropriate amine as starting material, and there were thus obtained as oils the compounds described in the following table, the structures of which were confirmed by elemental analysis and proton magnetic resonance spectroscopy.

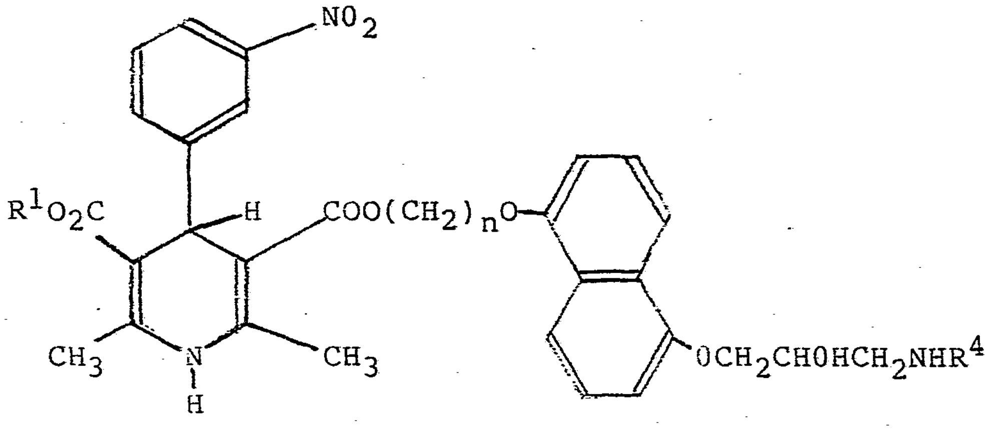

| $R^1$ | n | $R^4$ |
|---|---|---|
| methyl | 3 | isopropyl |
| ethyl | 4 | isopropyl |
| methyl | 5 | isopropyl |
| methyl | 5 | $(CH_3)_2CHCONH(CH_2)_2-$ |

The starting materials were prepared by a similar process to that described above using the appropriate alkylene dibromide and the appropriate pyridine-3-carboxylic acid.

Example 5

3-Bromopropyl 5-ethoxycarbonyl-1,4-dihydro-2,6-dimethyl 4-m-nitrophenylpyridine-3-carboxylate (1.5 g.) was added to a stirred solution of 1-p-hydroxyphenyl-2-isopropylaminoethanol (0.62 g.) and sodium hydride (0.15 g. of a 50% dispersion in mineral oil) in N,N-dimethylformamide (50 ml.) which had previously been stirred at laboratory temperature for 1 hour, and the mixture was stirred at laboratory temperature for 2 days and then evaporated to dryness under reduced pressure. The residue was dissolved in methylene chloride and the solution was washed with water, dried and evaporated to dryness. The residue was purified by flash chromatography on a silica gel (Merck 9385) column using methylene chloride containing

increasing amounts of methanol (up to 20% by volume) as eluant. There was thus obtained as an oil 3-p-(1-hydroxy-2-isopropylaminoethyl)phenoxypropyl 5-ethoxycarbonyl-1,4-dihydro-2,6-dimethyl-4-m-nitrophenylpyridine-3-carboxylate, the structure of which was confirmed by proton magnetic resonance spectroscopy in $CDCl_3$ as follows:-

| Shift (δ) | Type | No of protons | Assignment |
|---|---|---|---|
| 1.1-1.3 | triplet | 3 | $\underline{CH_3}CH_2$ |
| 1.3-1.5 | doublet | 6 | $(\underline{CH_3})_2CH$ |
| 1.9-2.1 | complex | 2 | $C-CH_2-C$ |
| 2.2-2.4 | singlet | 6 | $CH_3$ in pyridine |
| 2.9-3.1 | broad | 2 | $CH_2N$ |
| 3.1-3.5 | complex | 1 | $(CH_3)_2\underline{CH}$ |
| 3.7-3.8 | triplet | 2 | $CH_2$-O-aryl |
| 3.9-4.3 | complex | 4 | $CH_3\underline{CH_2}$,$CH_2OCO$ |
| 5.0-5.1 | complex | 2 | C$\underline{H}$OH, 4H of pyridine |
| 6.2 | singlet | 1 | pyridine NH |
| 6.6-8.1 | complex | 8 | aromatic |

The bromopropyl pyridine-3-carboxylate used as starting material was obtained as follows:-

Diketene (19.6 ml.) was added to stirred 3-bromopropanol (22.6 ml.) which was heated at 75°C., and the mixture was stirred at that temperature for 3 hours and then distilled under reduced pressure. There was thus obtained 3-bromopropyl acetoacetate, b.p. 83-86°C./0.2 mm.Hg.

A mixture of the above ester (22.3 g.), m-nitrobenzaldehyde (15.1 g.), ethyl aminocrotonate (12.8 g.) and isopropanol (100 ml.) was heated under

reflux for 4 hours, cooled and evaporated to dryness. The residue was stirred with diethyl ether and the mixture was filtered. There was thus obtained as solid residue the desired 3-bromopropyl 5-ethoxycarbonyl-1,4-dihydro-2,6-dimethyl-4-*m*-nitrophenylpyridine-3-carboxylate.

The process described in the first paragraph above was repeated using the appropriate haloalkyl pyridine-3-carboxylate and the appropriate *p*-hydroxyphenyl compound as starting materials, and there were thus obtained as oils the compounds described in the following table, the structures of all of which were confirmed by elemental analysis, mass spectroscopy and proton magnetic resonance spectroscopy.

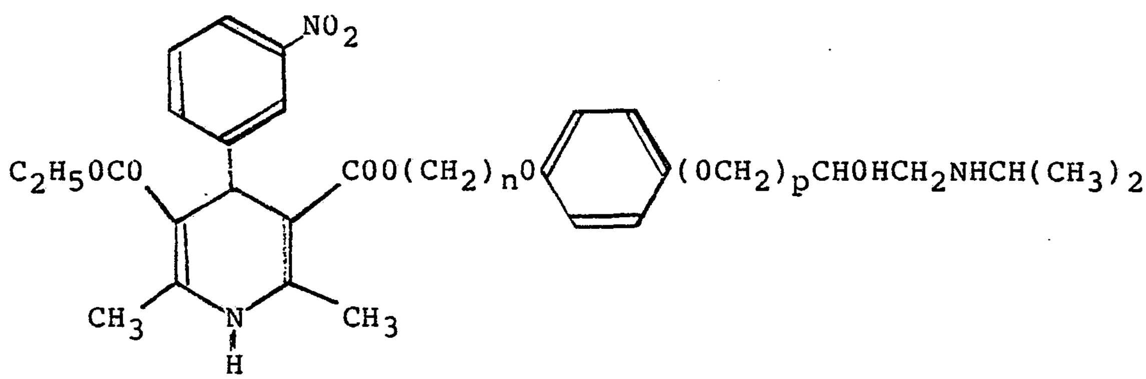

| n | p |
|---|---|
| 2 | 0 |
| 4 | 0 |
| 4 | 1 |

The haloalkyl piperidine-3-carboxylates were prepared by a similar process to that described in the second and third paragraphs above, using the appropriate bromoalkanol and ketene initially.

**What we claim is:**

1. A dihydropyridine of the formula:-

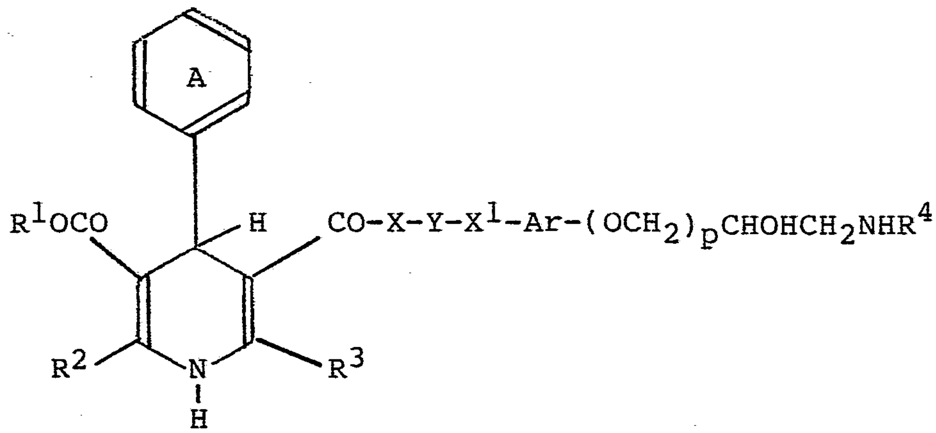

wherein $R^1$ is alkyl or alkoxyalkyl each of up to 6 carbon atoms:

wherein $R^2$ and $R^3$, which may be the same or different, each is alkyl of up to 6 carbon atoms;

wherein $R^4$ is alpha-branched-chain alkyl or hydroxyalkyl each of up to 6 carbon toms, or alpha-branched-chain arylalkyl or aryloxyalkyl each of up to 12 carbon atoms, or acylaminoalkyl wherein the acyl is of up to 10 carbon atoms and the alkyl is of up to 6 carbon atoms;

wherein benzene ring A bears one or more substituents selected from halogeno, cyano, nitro, trifluoromethyl and alkyl of up to 6 carbon atoms or bears the substituent

=N-O-N=

attached to the 2- and 3-positions (that is, to form a benzo-2,1,3-oxadiazole nucleus);

wherein Ar is phenylene, naphthylene, tetrahydronaphthylene, indanylene or pyridylene which is unsubstituted or which bears one or more substituents selected from halogeno, trifluoromethyl, hydroxy, amino,

nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;
wherein p is 0 or 1;
wherein X is -O- or -NH-;
wherein $X^1$ is a direct link or is -O-, -S-, -NH or $-NHSO_2-$;
and wherein Y is straight-or branched-chain alkylene of 1 to 12 carbon atoms which may optionally be interrupted by one or two groups selected from oxygen (-O-), sulphur (-S-), imino and substituted imino ($-NR^5$ wherein $R^5$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,3- or 1,4-piperidinediyl and amido (-CONH-or -NHCO-) groups;
or an acid-addition salt thereof.

2. A dihydropyridine as claimed in claim 1 wherein $R^1$ is methyl or ethyl, $R^2$ and $R^3$ are both methyl, $R^4$ is isopropyl, t-butyl, 2-hydroxy-1-methylethyl, 2-hydroxy-1,1-dimethylethyl or 2-isobutyramidoethyl, benzene ring A is 3-nitrophenyl, 2-chlorophenyl or 2,3-dichlorophenyl, Ar is 1,4-phenylene or 1,2-phenylene which may contain a chloro or methoxy substituent, or is 1,5-naphthalene, p is 0 or 1, X is -O-, Y is $-(CH_2)_n-$which may optionally be interrupted by -O- and/or -NHCO-, and $X^1$ is a direct link or -O-.

3. A dihydropyridine as claimed in claim 2 wherein $-YX^1-$ is $-(CH_2)_n-O-$ or $-(CH_2)_mNHCOCH_2-$ wherein n is 2, 3, 4, 5, 6 or 7 and m is 2, 3, 4, 5 or 6, or is

$-(CH_2)_2-O-(CH_2)_2NHCOCH_2-$.

4. The compound 3-[p-(2-hydroxy-3-isopropylaminopropoxy)phenylacetamido] propyl 5-ethoxycarbonyl-1,4-dihydro-2,6-dimethyl-4-m-nitrophenylpyridine-3-carboxylate.

5. The compound:-

3-[p-(2-hydroxy-3-isopropylaminopropoxy)-phenoxy]propyl 5-ethoxycarbonyl-1,4-dihydro-2,6-dimethyl-4-m-nitrophenylpyridine-3-carboxylate and the corresponding 4-[p-(2-hydroxy-3-isopropylaminopropoxy)-phenoxy]butyl, 5-[p-(2-hydroxy-3-isopropylamino-propoxy)phenoxy]pentyl, 6-[p-(2-hydroxy-3-isopropyl-aminopropoxy)phenoxy]hexyl and 5-[4-(2-hydroxy-3-isopropylaminopropoxy)-3-methoxyphenoxy]pentyl esters;

3-[5-(2-hydroxy-3-isopropylaminopropoxy)-naphth-1-yloxy]propyl 1,4-dihydro-5-methoxycarbonyl-2,6-dimethyl-4-m-nitrophenylpyridine-3-carboxylate and the corresponding 5-[5-(2-hydroxy-3-isopropylaminopropoxy)-naphth-1-yloxy]pentyl ester;

4-[5-(2-hydroxy-3-isopropylaminopropoxy)-naphth-1-yloxy]butyl 5-ethoxycarbonyl-1,4-dihydro-2,6-dimethyl-4-m-nitrophenylpyridine-3-carboxylate;

2-[p-(1-hydroxy-2-isopropylaminoethyl)-phenoxy]ethyl 5-ethoxycarbonyl-1,4-dihydro-2,6-dimethyl-4-m-nitrophenylpyridine-3-carboxylate and the corresponding 3-[p-(1-hydroxy-2-isopropylaminoethyl)-phenoxy]propyl ester;

4-[p-(1-hydroxy-2-isopropylaminoethyl)-phenoxy]butyl 1,4-dihydro-5-methoxycarbonyl-2,6-dimethyl-4-m-nitrophenylpyridine-3-carboxylate; or

1-[p-(2-hydroxy-3-isopropylaminopropoxy)-benzyl]piperidin-3-yl 1,4-dihydro-5-methoxycarbonyl-2,6-dimethyl-4-m-nitrophenylpyridine-3-carboxylate.

6. A process for the manufacture of a dihydropyridine, claimed in any of claims 1 to 5 which comprises:-

(a) the reaction of an epoxide of the formula:-

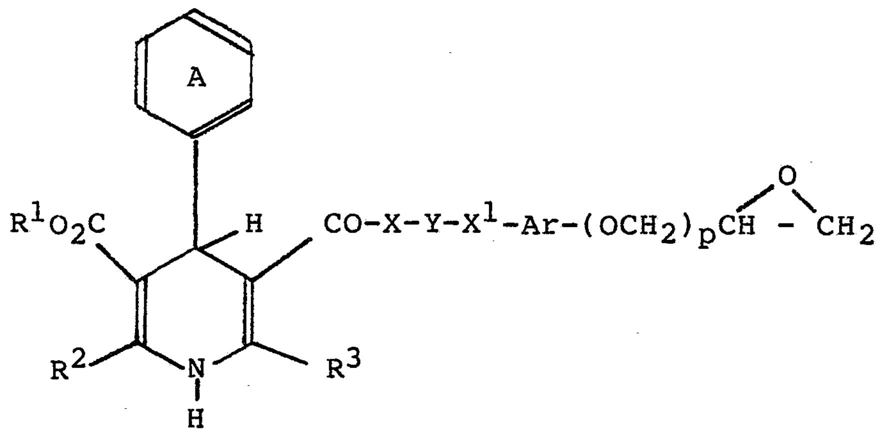

wherein A, $R^1$, $R^2$, $R^3$, X, $X^1$, Y, Ar and p have the meanings stated in claim 1, 2 or 3 with an amine of the formula:-

$$R^4NH_2$$

wherein $R^4$ has the meaning stated in claim 1 or 2, or when p is 0, the reaction of said amine with a haloketone of the formula

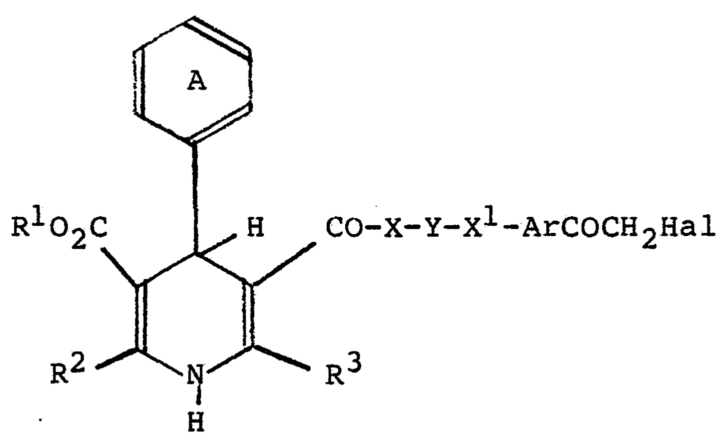

wherein A, $R^1$, $R^2$, $R^3$, X, $X^1$, Y and Ar have the meanings stated above and wherein Hal stands for a halogeno group, followed by reduction of the aminoketone thus obtained; or

(b) for the manufacture of a dihydropyridine wherein the group -Y- is alkylene interrupted by an amido group -NHCO-, reactions of an amine of the formula:-

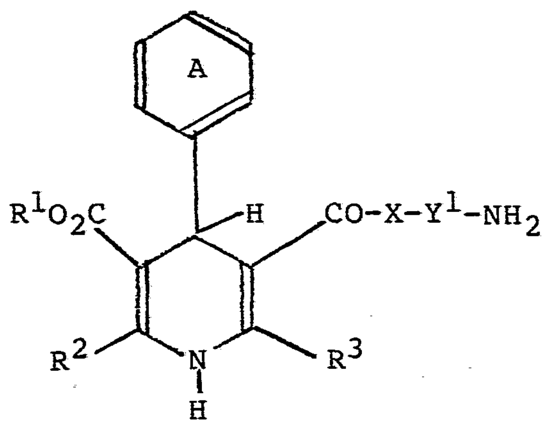

wherein A, $R^1$, $R^2$, $R^3$ and X have the meanings stated above, with an acid of the formula:-

$$HO_2C-Y^2-X^1-Ar-(OCH_2)_pCHOHCH_2NHR^4$$

wherein $X^1$, Ar, p and $R^4$ have the meanings stated above, or with an activated derivative thereof, and wherein $Y^1$ and $Y^2$ are such that $-Y^1-NHCO-Y^2-$ has the same meaning as stated above for Y; or

(c) the reaction of an acid of the formula:-

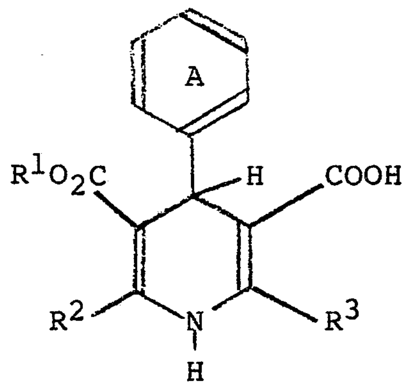

wherein A, $R^1$, $R^2$ and $R^3$ have the meanings stated above, or an activated derivative thereof, with an alcohol or amine of the formula:-

$$H-X-Y-X^1-Ar(OCH_2)_pCHOHCH_2NHR^4$$

wherein $R^4$, Ar, p, X, $X^1$ and Y have the meanings stated above; or

(d) for the manufacture of a dihydropyridine wherein $X^1$ is other than a direct link, the reaction of a compound of the formula:-

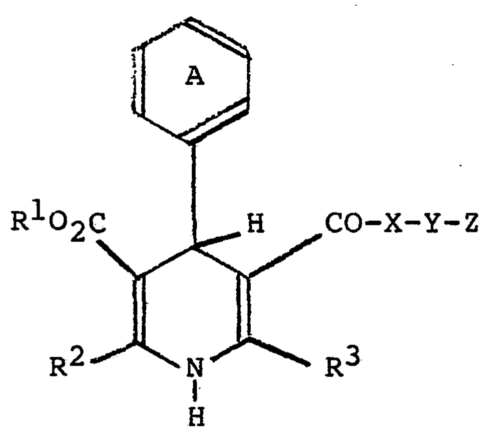

wherein A, $R^1$, $R^2$, $R^3$, X and Y have the meanings stated above and wherein Z stands for a displaceable group, with a compound of the formula:-

$$H-X^1-Ar(OCH_2)_pCHOHCH_2NHR^4$$

wherein $X^1$,Ar,p and $R^4$ have the meanings stated above; or

(e) the reaction of an aldehyde of the formula

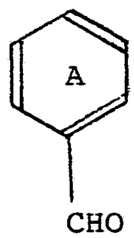

wherein A has the meaning stated above, an aminocrotonate of the formula

$$R^2-\overset{NH_2}{\overset{|}{C}}=CH-CO_2R^1$$

wherein $R^1$ and $R^2$ have the meanings stated above and a ketoacid derivative of the formula

$$R^3COCH_2CO-X-Y-X^1-Ar(OCH_2)_pCHOHCH_2NHR^4$$

wherein Ar, p, $R^3$, $R^4$, X and Y have the meanings stated above.

7. A pharmaceutical composition comprising as active ingredient at least one dihydropyridine, claimed in any of claims 1 to 5, or an acid-addition salt thereof, in association with a pharmaceutically-acceptable diluent or carrier therefor.

8. A composition as claimed in claim 7 which is a tablet, capsule, aqueous or oily solution or suspension, dispersible powder, spray or aerosol formulation.

9. A composition as claimed in claim 7 or 8 which contains, in addition to the dihydropyridine, one or more drugs selected from sedatives, vasodilators,

diuretics, other antihypertensive agents, cardiac membrane stabilising agents, agents used in the treatment of Parkinson's disease and other tremors, cardiotonic agents, and alpha-adrenergic blocking agents.

10. The use of a compound, claimed in any of claims 1 to 5, for the manufacture of a medicament for producing an antihypertensive effect in a warm-blooded animal.

REGINALD PETER SLATCHER

AUTHORISED REPRESENTATIVE
General Authorisation No. 97

SE01
PS33366
RPS/KEB: 28 JAN 86

## Claims for Austria

What we claim is:

1. A process for the manufacture of a dihydropyridine of the formula:-

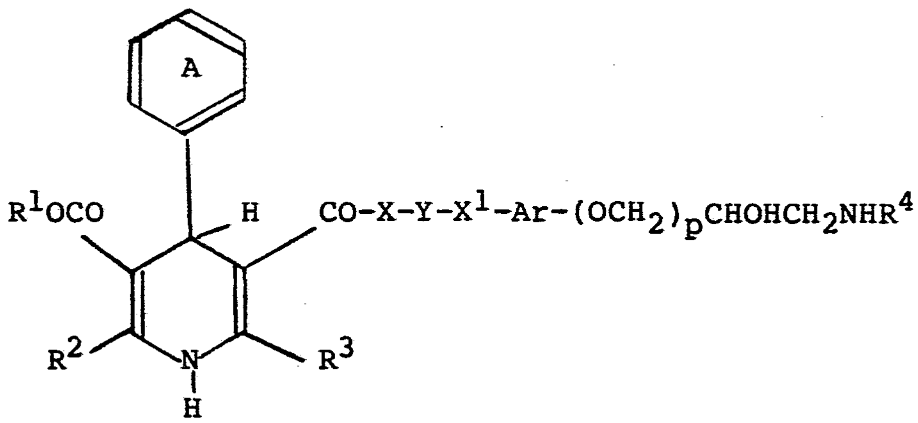

wherein $R^1$ is alkyl or alkoxyalkyl each of up to 6 carbon atoms:
wherein $R^2$ and $R^3$, which may be the same or different, each is alkyl of up to 6 carbon atoms;
wherein $R^4$ is alpha-branched-chain alkyl or hydroxyalkyl each of up to 6 carbon toms, or alpha-branched-chain arylalkyl or aryloxyalkyl each of up to 12 carbon atoms, or acylaminoalkyl wherein the acyl is of up to 10 carbon atoms and the alkyl is of up to 6 carbon atoms;
wherein benzene ring A bears one or more substituents selected from halogeno, cyano, nitro, trifluoromethyl and alkyl of up to 6 carbon atoms or bears the substituent

$$=N-O-N=$$

attached to the 2- and 3-positions (that is, to form a benzo-2,1,3-oxadiazole nucleus);
wherein Ar is phenylene, naphthylene, tetrahydronaphthylene, indanylene or pyridylene which is unsubstituted or which bears one or more substituents selected from halogeno, trifluoromethyl, hydroxy, amino,

nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;

wherein p is 0 or 1;

wherein X is -O- or -NH-;

wherein $X^1$ is a direct link or is -O-, -S-, -NH or $-NHSO_2-$;

and wherein Y is straight-or branched-chain alkylene of 1 to 12 carbon atoms which may optionally be interrupted by one or two groups selected from oxygen (-O-), sulphur (-S-), imino and substituted imino ($-NR^5$ wherein $R^5$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,3- or 1,4-piperidinediyl and amido (-CONH-or -NHCO-) groups;

or an acid-addition salt thereof, characterised by:-

(a) the reaction of an epoxide of the formula:-

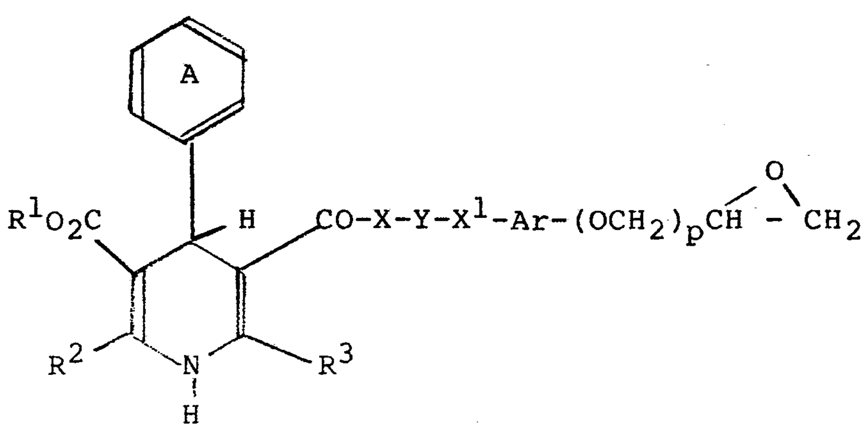

wherein A, $R^1$, $R^2$, $R^3$, X, $X^1$, Y, Ar and p have the meanings stated above, with an amine of the formula:-

$R^4NH_2$

wherein $R^4$ has the meaning stated above, or when p is 0, the reaction of said amine with a haloketone of the formula

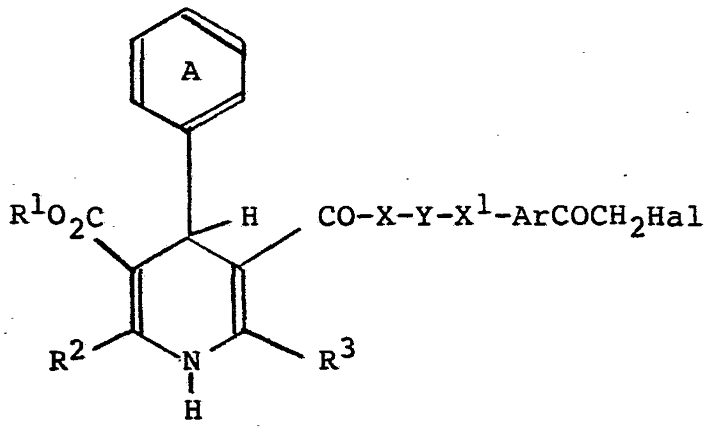

wherein A, $R^1$, $R^2$, $R^3$, X, $X^1$, Y and Ar have the meanings stated above and wherein Hal stands for a halogeno group, followed by reduction of the aminoketone thus obtained; or

(b) for the manufacture of a dihydropyridine wherein the group -Y- is alkylene interrupted by an amido group -NHCO-, reactions of an amine of the formula:-

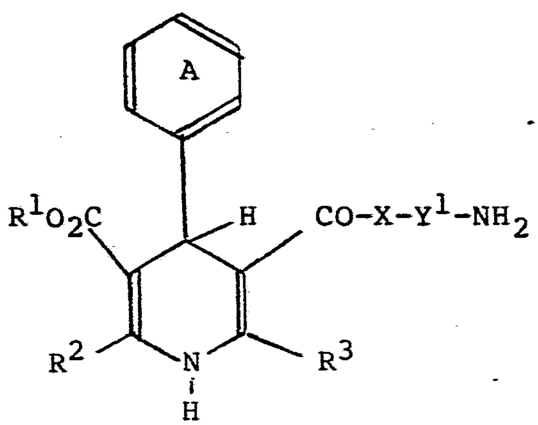

wherein A, $R^1$, $R^2$, $R^3$ and X have the meanings stated above, with an acid of the formula:-

$$HO_2C-Y^2-X^1-Ar-(OCH_2)_pCHOHCH_2NHR^4$$

wherein $X^1$, Ar, p and $R^4$ have the meanings stated above, or with an activated derivative thereof, and wherein $Y^1$ and $Y^2$ are such that $-Y^1-NHCO-Y^2-$ has the same meaning as stated above for Y; or

(c) the reaction of an acid of the formula:-

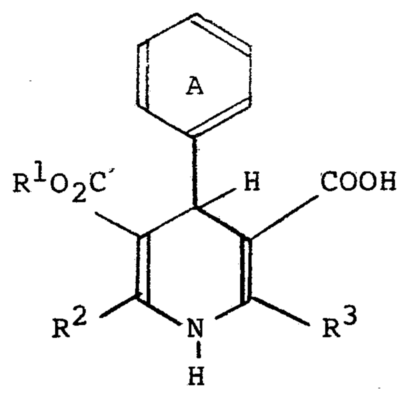

wherein A, $R^1$, $R^2$ and $R^3$ have the meanings stated above, or an activated derivative thereof, with an alcohol or amine of the formula:-

$$H-X-Y-X^1-Ar(OCH_2)_pCHOHCH_2NHR^4$$

wherein $R^4$, Ar, p, X, $X^1$ and Y have the meanings stated above; or

(d) for the manufacture of a dihydropyridine wherein $X^1$ is other than a direct link, the reaction of a compound of the formula:-

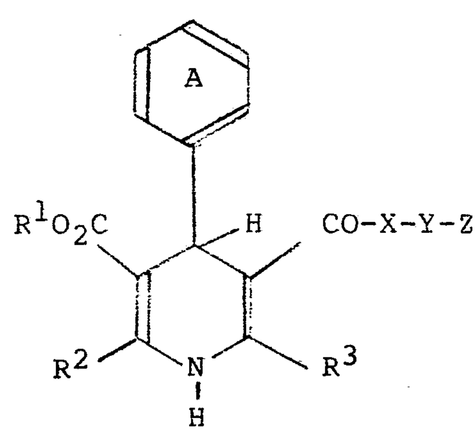

wherein A, $R^1$, $R^2$, $R^3$, X and Y have the meanings stated above and wherein Z stands for a displaceable group, with a compound of the formula:-

$$H-X^1-Ar(OCH_2)_pCHOHCH_2NHR^4$$

wherein $X^1$,Ar,p and $R^4$ have the meanings stated above; or

(e) the reaction of an aldehyde of the formula

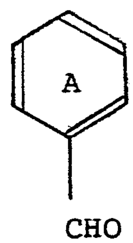

wherein A has the meaning stated above, an aminocrotonate of the formula

$$R^2-\overset{NH_2}{\overset{|}{C}}=CH-CO_2R^1$$

wherein $R^1$ and $R^2$ have the meanings stated above and a ketoacid derivative of the formula

$$R^3COCH_2CO-X-Y-X^1-Ar(OCH_2)_pCHOHCH_2NHR^4$$

wherein Ar, p, $R^3$, $R^4$, X and Y have the meanings stated above.

2. A process as claimed in claim 1 wherein in the starting materials $R^1$ is methyl or ethyl, $R^2$ and $R^3$ are both methyl, $R^4$ is isopropyl, t-butyl, 2-hydroxy-1-methylethyl, 2-hydroxy-1,1-dimethylethyl or 2-isobutyramidoethyl, benzene ring A is 3-nitrophenyl, 2-chlorophenyl or 2,3-dichlorophenyl, Ar is 1,4-phenylene

or 1,2-phenylene which may contain a chloro or methoxy substituent, or is 1,5-naphthalene, p is 0 or 1, X is -0-, Y is $-(CH_2)_n-$which may optionally be interrupted by -0- and/or -NHC0-, and $X^1$ is a direct link or -0-.

3. A process as claimed in claim 2 wherein in the starting materials $-YX^1-$is $-(CH_2)_n-0-$ or $-(CH_2)_mNHCOCH_2-$ wherein n is 2, 3, 4, 5, 6 or 7 and m is 2, 3, 4, 5 or 6, or is $-(CH_2)_2-0-(CH_2)_2NHCOCH_2-$.

REGINALD PETER SLATCHER

AUTHORISED REPRESENTATIVE
General Authorisation No. 97

PS33366/AT
SE04:
RPS/KEB: 6 Jan 86

0194046

European Patent Office

# EUROPEAN SEARCH REPORT

Application number

EP 86 30 0885

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | JOURNAL OF MEDICINAL CHEMISTRY, vol. 24, no. 5, May 1981, pages 628-631, American Chemical Society, Washington, US; J.J. BALDWIN et al.: "Approaches to vasodilating/bêta-adrenergic blocking agents: examples of the dihydrolutidine type" * Page 629, compounds 4a,b,c; page 631, experimental section; page 630-631, discussion * | 1-3,6-10 | C 07 D 211/90<br>C 07 D 401/12<br>A 61 K 31/44 |
| A | GB-A-1 409 865 (SCIENCE UNION) * Example X; claims * | 1-3,6-10 | |
| P,A | CHEMICAL ABSTRACTS, vol. 104, no. 13, 31st March 1986, page 711, no. 109478d, Columbus, Ohio, US; & JP - A - 60 136 558 (TEIKOKU HORMONE MFG. CO.) 20-07-1985 | 1-3,6-10 | **TECHNICAL FIELDS SEARCHED (Int. Cl.4)** |
| P,A | EP-A-0 151 006 (YAMANOUCHI PHARMACEUTICAL CO., LTD.) * Whole document, especially examples 1-58 * | 1-3,6-10 | C 07 D 211<br>C 07 D 401<br>A 61 K 31 |

The present search report has been drawn up for all claims

| Place of search | Date of completion of the search | Examiner |
|---|---|---|
| THE HAGUE | 26-05-1986 | NUYTS A.M.K.A. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document